# EUROPEAN PATENT SPECIFICATION

(11) **EP 0 537 846 B1**
(45) Date of publication and mention of the grant of the patent: **27.03.1996**
(21) Application number: 92203109.1
(22) Date of filing: 08.10.1992
(51) Int. Cl.: C07C 31/36, C07C 29/66

(54) **Production of dichlorohydrin**
Herstellung von Dichlorhydrin
Production de dichlorohydrine

(30) Priority: 10.10.1991 EP 91202641
(43) Date of publication of application: 21.04.1993
(73) Proprietor: SHELL INTERNATIONALE RESEARCH MAATSCHAPPIJ B.V., NL-2596 HR Den Haag (NL)
(72) Inventor: John, Christopher Stephen, NL-1031 CM Amsterdam (NL); Maas, Wilfridus Petrus Maria, NL-1031 CM Amsterdam (NL)

(56) References cited:
- EP-A- 0 359 331

## Description

The invention relates to a process for the production of dichlorohydrin and to dichlorohydrin prepared by such process. Dichlorohydrin is a term employed herein to designate the isomers 1,2-dichloro-3-hydroxypropane and 1,3-dichloro-2-hydroxypropane.

It is known to prepare dichlorohydrin by reacting in a reaction zone allylchloride, water and chlorine in a dilute aqueous phase.

A problem with the preparation of dichlorohydrin is that other chlorinated organic compounds are formed as well, as byproducts. Besides large amounts of water are necessary in the reaction which means that large effluent streams exist which pose a problem for the environment. Most chlorinated organic byproducts are toxic, non-biodegradable and non-hydrolysable.

A process has been found to reduce the level of these impurities in the dichlorohydrin.

The invention relates to a process for the production of dichlorohydrin which comprises reacting allylchloride, water and chlorine in a reaction zone to form an aqueous dichlorohydrin solution together with other chlorinated organic byproducts, and contacting the aqueous reaction zone effluent with 1,2,3-trichloropropane to obtain an aqueous layer comprising the dichlorohydrin and an organic layer comprising the trichloropropane and said chlorinated organic byproducts, separating both layers and if desired working up the dichlorohydrin containing aqueous solution in a manner known per se.

From European Patent Application Publication 359.331 is known a process for the preparation of dichlorohydrin wherein allylchloride, water and chlorine are reacted in a reaction zone to form an aqueous dichlorohydrin solution and thereafter extracting the aqueous dichlorohydrin solution with a water-immiscible solvent having an atmospheric boiling point from 40 °C to 105 °C in order to remove chlorinated organic impurities.

The water-immiscible solvents have a rather high vapour pressure compared with the vapour pressure of 1,2,3-trichloropropane. The latter compound has a boiling point of 156 °C at atmospheric pressure.

Besides 1,2,3-trichloropropane has the advantage that it is not a compound which is alien to the dichlorohydrin preparation process. Namely it is found as a byproduct of the dichlorohydrin preparation.

The amount of 1,2,3-trichloropropane used is usually less than 10 per cent by weight on the weight of the aqueous dichlorohydrin solution, preferably from 1 to 8 per cent, more preferably from 1.5 to 5 per cent, most preferably from 2 to 4 per cent by weight on the weight of the aqueous dichlorohydrin solution (that is the reaction zone effluent). The used 1,2,3-trichloropropane may be added or is already present in the aqueous dichlorohydrin solution.

The 1,2,3-trichloropropane may be contacted with the aqueous dichlorohydrin solution in a cocurrent stream or in a counter-current stream.

The process for the preparation of dichlorohydrin may be carried out batchwise, semi-continuously or continuously. The same applies to the extraction with 1,2,3-trichloropropane.

Once that the aqueous dichlorohydrin solution has been treated with 1,2,3-trichloropropane and the byproducts (impurities) have been removed from the aqueous dichlorohydrin solution, the dichlorohydrin is worked up in a manner known per se, e.g. by distillation or the aqueous dichlorohydrin solution is directly used in the preparation of epichlorohydrin. The advantage is that the content of the byproducts from the dichlorohydrin preparation is reduced. This in turn results in an improvement in the quality of the aqueous effluent streams.

The 1,2,3-trichloropropane containing the chlorinated organic byproducts may be conducted to a distillation zone, wherein 1,2,3-trichloropropane and lighter products are distilled overhead and chlorinated organic byproducts are recovered as bottom products. The bottom products may be incinerated.

The distilled 1,2,3-trichloropropane may be recycled to the extraction zone.

The extraction solvent 1,2,3-trichloropropane contains a certain amount of dichlorohydrin. This product can be distilled in the distillation zone overhead together with the 1,2,3-trichloropropane and recycled to the extraction zone. The 1,2,3-trichloropropane is also built up in the system and therefore a bleed stream is necessary.

Another embodiment of the invention comprises a process wherein no 1,2,3-trichloropropane is added. In that case the reaction zone effluent is allowed to settle and a heavy organic layer comprising 1,2,3-trichloropropane (as reaction byproduct) is separated from the aqueous effluent.

### Example

A continuous stream of aqueous dichlorohydrin solution, obtained by reaction of allylchloride, water and chlorine, and 1,2,3-trichloropropane were fed through a liquid-liquid mixer to a hydrocyclone. Per kilogram of aqueous dichlorohydrin solution 25 g of added 1,2,3-trichloropropane was used. The hydrocyclone separated the mixture into an aqueous phase, comprising the dichlorohydrin, and organic phase comprising the 1,2,3-trichloropropane.

The extraction efficiency of 1,2,3-trichloropropane was studied by analysis of hexane layers of 1:1 volume hexane extracts of the aqueous solutions of dichlorohydrin prior to and after extraction with 1,2,3-trichloropropane.

The analysis of the hexane layers took place with a gas chromatograph equipped with a HALL-detector which gave a signal for chlorine and linear with respect to the chlorine load of the components. The total chlorine content was obtained by summation of the detector response for all chlorinated components present in the hexane extracts with a higher boiling point than that of dichlorohydrin (1,2-dichloro-3-hydroxypropane, b.p. 183 °C). The extraction efficiency is the reduction of the measured chlorine content of the hexane layers obtained prior to and after treatment with 1,2,3-trichloropropane.

The extraction efficiency of 1,2,3-trichloropropane was 90%.

## Claims

1. A process for the production of dichlorohydrin which comprises reacting allylchloride, water and chlorine in a reaction zone to form an aqueous dichlorohydrin solution together with other chlorinated organic byproducts, and contacting the aqueous reaction zone effluent with 1,2,3-trichloropropane to obtain an aqueous layer comprising the dichlorohydrin and an organic layer comprising the trichloropropane and said chlorinated organic byproducts, separating both layers and if desired working up the dichlorohydrin containing aqueous solution in a manner known per se.

2. A process as claimed in claim 1, wherein less than 10 per cent by weight of 1,2,3-trichloropropane is used on the weight of the effluent.

3. A process as claimed in claim 2, wherein from 1 to 8 per cent by weight of 1,2,3-trichloropropane is used on the weight of the effluent.

4. A process as claimed in claims 1-3, wherein the 1,2,3-trichloropropane is contacted with the aqueous dichlorohydrin solution cocurrently or countercurrently.

5. A process as claimed in any one of the claims 1-4, wherein the organic layer containing trichloropropane and chlorinated organic byproducts is conducted to a distillation zone, wherein the trichloropropane is distilled (over the top) and the chlorinated organic byproducts remain as bottom products.

6. A process as claimed in claim 5, wherein a part of the distilled trichloropropane is recycled to the extraction zone.

7. A process as claimed in claim 1, wherein the reaction zone effluent is allowed to settle and a heavy organic layer comprising trichloropropane is separated from the aqueous effluent, directly after the reaction.

## Patentansprüche

1. Verfahren zur Herstellung von Dichlorhydrin, bei dem man Allylchlorid, Wasser und Chlor in einer Reaktionszone zur Erzeugung einer wäßrigen Lösung von Dichlorhydrin sowie anderen chlorierten organischen Nebenprodukten zur Reaktion bringt und den Austrag der wäßrigen Reaktionszone mit 1,2,3-Trichlorpropan in Berührung bringt, wobei man eine wäßrige, das Dichlorhydrin enthaltende Schicht und eine organische, das Trichlorpropan und besagte chlorierte organische Nebenprodukte enthaltende Schicht erhält, die beiden Schichten voneinander trennt und gewünschtenfalls die Dichlorhydrin enthaltende wäßrige Lösung in an sich bekannter Weise aufarbeitet.

2. Verfahren nach Anspruch 1, bei dem man weniger als 10 Gew.-% 1,2,3-Trichlorpropan, bezogen auf das Austragsgewicht, verwendet.

3. Verfahren nach Anspruch 2, bei dem man 1 bis 8 Gew.-% 1,2,3-Trichlorpropan, bezogen auf das Austragsgewicht, verwendet.

4. Verfahren nach den Ansprüchen 1-3, bei dem man das 1,2,3-Trichlorpropan mit der wäßrigen Dichlorhydrinlösung im Gleichstrom oder im Gegenstrom in Berührung bringt.

5. Verfahren nach einem der Ansprüche 1-4, bei dem man die organische, Trichlorpropan sowie chlorierte organische Nebenprodukte enthaltende Schicht in eine Destillationszone überführt, in der man das Trichlorpropan über Kopf abdestilliert und die chlorierten organischen Nebenprodukte als Sumpfprodukte zurückbleiben.

6. Verfahren nach Anspruch 5, bei dem man einen Teil des destillierten Trichlorpropans in die Extraktionszone zurückführt.

7. Verfahren nach Anspruch 1, bei dem man den Austrag der Reaktionszone absetzen läßt und eine schwere organische, das Trichlorpropan enthaltende Schicht vom wäßrigen Austrag sofort nach der Reaktion abtrennt.

## Revendications

1. Procédé de production de la dichlorhydrine, caractérisé en ce que l'on fait réagir du chlorure d'allyle, de l'eau et du chlore dans une zone de réaction pour former une solution aqueuse de dichlorhydrine, en même temps que d'autres sous-produits organiques chlorés et on met l'effluent ou produit quittant la zone de réaction aqueux en contact avec du 1,2,3-trichloropropane pour obtenir une couche aqueuse contenant la dichlorhydrine et une couche organique comprenant le trichloropropane et lesdits sous-produits organiques chlorés, on sépare les deux couches et, si cela se révèle souhaitable, on traite la solution aqueuse contenant la dichlorhydrine d'une manière en soi connue.

2. Procédé suivant la revendication 1, caractérisé en ce que l'on utilise moins de 10% en poids de 1,2,3-trichloropropane par rapport au poids de l'effluent.

3. Procédé suivant la revendication 2, caractérisé en ce que l'on utilise de 1 à 8% en poids de 1,2,3-trichloropropane par rapport au poids de l'effluent.

4. Procédé suivant l'une quelconque des revendications 1 à 3, caractérisé en ce que l'on met le 1,2,3-trichloropropane en contact avec la solution aqueuse de dichlorhydrine en équicourant ou en contre-courant.

5. Procédé suivant l'une quelconque des revendications 1 à 4, caractérisé en ce que l'on amène la couche organique contenant du trichloropropane et des sous-produits organiques chlorés à une zone de distillation, dans laquelle le trichloropropane est distillé (en tête) et les sous-produits organiques chlorés subsistent sous forme de produits de fond ou de queue.

6. Procédé suivant la revendication 5, caractérisé en ce que l'on renvoie une partie du trichloropropane distillé à la zone d'extraction.

7. Procédé suivant la revendication 1, caractérisé en ce qu'on laisse décanter l'effluent de la zone de réaction et on sépare une couche organique lourde comprenant du trichloropropane de l'effluent aqueux, directement après la réaction.
